# EUROPÄISCHE PATENTANMELDUNG

(11) **EP 1 270 587 A1**
(43) Veröffentlichungstag der Anmeldung: **02.01.2003**
(21) Anmeldenummer: 01115083.6
(22) Anmeldetag: 21.06.2001
(51) Int. Cl.: C07K 14/415, C12N 15/82, A01H 5/00, C12N 15/11, C12N 5/10, C12N 15/29

(54) **Transportproteine für Oxoderivate stickstoffhaltiger Heterozyklen und hierfür codierende Nukleinsäuren**

(71) Anmelder: Frommer, Wolf-Bernd, Prof. Dr., 72076 Tübingen (DE)
(72) Erfinder: Schumacher, Karin Dr., 71070 Tübingen (DE); Desimone, Marcelo Dr., 79224 Umkirch (DE); Catoni Müller, Elisabetta Dr., 70186 Stuttgart (DE); Hilpert, Melanie, 72108 Rottenburg (DE); Frommer, Wolf B. Prof. Dr., 72076 Tübingen (DE)
(74) Vertreter: Pohl, Manfred, Dr.

(57) **Zusammenfassung**

Die Erfindung betrifft Nukleinsäuren, die die codierende Region für ein Transportprotein für stickstoffhaltige Heterozyklen, insbesondere deren Oxoderivate, enthalten. Die Erfindung stellt Nukleinsäuren, entsprechende Transportproteine sowie ein Verfahren zur Transformation von Pflanzen mit den erfindungsgemäßen Nukleinsäuren zur Verfügung, um eine gezielte Kontrolle des Im- und Exports von N-haltigen Heterozyklen, insbesondere von Allantoin, bei Pflanzen zu ermöglichen.

SEQUENZPROTOKOLL

<110> Frommer, Wolf B.

<120> Transportproteine und hierfür codierende Nukleinsäuren

<130> P 749

<140>
<141>

<160> 22

<170> PatentIn Ver. 2.1

<210> 1
<211> 1173
<212> DNA
<213> Arabidopsis thaliana

<220>
<223> UPS1-Gen

<400> 1
atgtatatga tagagagcaa aggaggagca attgcatgta tgctcttggc tcttctcttc 60
ttagggacat ggccagcaat catgactcta accgaaagac gcggtcgact tcctcaacac 120
acttatcttg actacacact cacaaatctg ttagctgcag tgatcatagc gttgacacta 180
ggagaaatag gtccgagcag accaaatttc tttacacagc tctctcaaga taattggcag 240
tcagtgatgt ttgcaatggc tggaggaata gttcttagcc ttggaaactt agcaacacaa 300
tatgcttggg cttatgttgg tttatcagtt actgaagtca tcacagctag tatcactgtt 360
gttatcggca caactttgaa ctatttcttg gatgatagaa tcaacagagc tgaggtcctt 420
ttcccggggg tcgcttgttt cttgatcgct gtttgtttcg gttccgctgt tcataaatcc 480
aatgctgctg ataacaaaac caaactccaa aatttcaaaa gtttagagac tacttcttcc 540
ttcgaaatgg aaacaatttc tgcaagcaac gggttaacaa aagggaaagc gaaagaaggg 600
actgcagctt ttctcataga gcttgagaag caaagagcta taaaggtctt tggcaaaagc 660
acaataatcg gattggtgat aactttcttt gccggtatct gcttttctct gttctcacct 720

gcattcaact tagcaacaaa cgatcaatgg cacacattaa aacatggagt tccaaaacta 780
aatgtgtaca ctgccttctt ctacttctcc atctctgcat ttgtagttgc tctgatactt 840
aacatcagat ttctctattg gcctatactt ggtctcccaa gatcttcttt caaggcttat 900
ttgaatgact ggaacggtcg aggctggtct ttcttggctg gatttctatg tggatttggc 960
aatggtcttc agttcatggg tggtcaagcc gcaggctatg cagctgcaga cgctgttcag 1020
gcacttccac ttgtgagtac attttggggg atattgttgt ttggagaata tagaagatca 1080
tcaagaaaaa catatacact tctcatcagt atgcttctta tgttcatagt cgctgtcgcg 1140
gttcttatgg cttcctcggg acatagaaaa tga 1173

<210> 2
<211> 1158
<212> DNA
<213> Arabidopsis thaliana

<220>
<223> UPS2-Gen

<400> 2
atgcttttag ccttactatc tctagggact tggccagcag ttttaacact tttagagaga 60
cgtggccgtc ttcctcaaca cacctatctt gattactcca tcactaactt gttggctgct 120
attattatag ctttcacttt tggtcaaatt gggtctacaa aacccgatag cccaaacttc 180
atcactcagc ttgctcaaga taattggccg tctgttatgt ttgcaatggc gggcggtatc 240
gtgcttagcc tcgggaatct atcaactcaa tatgcatggg ctttagtagg cttatcagtt 300
acagaagtca tcacatcaag catcactgta gtcataggct caaccttgaa ctatttcttg 360
gatgataaga tcaacaaagc cgagatcctt ttccccggtg tggcctgttt cttgatcgcg 420
gtttgtcttg gttcagcagt tcatagatcc aatgcagacg ataacaaggc caagctaaga 480
gactttgaaa cggctaagca agaagcttca ggtccatcta ccgaaatagg aacaaactca 540
tcaaaagacc tggagactaa tgttacgaca aaacctaaag aagggacagc aagatttctt 600
atagagcttg agaacacaag agccataaag gtgttcggaa aacgaaagat aatcggacta 660
gcgattactt tcttcgcagg tctttgtttc tccctcttct ctccagcatt taacttagcc 720
actaacgatc aatggaacag attgaaacaa ggagtgccaa agctagtcgt ttacacagca 780
ttcttctact tctctgtttc ttgttttatc attgcattga tactcaacgt tgtattcctc 840
tactatcctg ttcttggtct tccaaaatct tcttttaaag cttacttaaa tgactggaat 900
ggtcgttact gggccttttt ggcggggttt ctttgcggtt ttggaaacgg tcttcagttt 960
atgggcggtc aagccgcagg atacgcggct gctgactccg ttcaggctct tccacttgtg 1020

agtacatttt ggggagtggt tttgtttgga gaatacagaa gatcgtcacg gaaaacttat 1080
ctacttctct tctgtatgtt gtttatgttc atttcagctg ttgcagttct catggcttct 1140
tctggacata gaaagtaa 1158

<210> 3
<211> 1035
<212> DNA
<213> Arabidopsis thaliana

<220>
<223> UPS3-Gen

<400> 3
atgtatgtga tagagagcaa aggaggaaca atcacttgta tgctcttggc tctgctcttc 60
ttagggacat ggccagcaat catgactcta accgaaagac gcggtagact tcctcaacat 120
acttatcttg actacacact gacaaacctg ttagctgcag ttatcatagc gtttacacta 180
ggcgaaataa gtccaagcag accaaatttc accacacagc tttctcaaga taattggccg 240
tctgtgatgt ttgcaatggc tggaggaata tttcttagcc ttgggacctt agcaacacaa 300
tatgcttggg cttttgttgg gttatctgtc actgaagtca tcacagctag tatcgccgtt 360
gttatcggca caactttgaa ctatttcttg gatgatagaa tcaacagagc tgaggtcctt 420
ttcccggggg tcgcttgttt cttgatcgct gtttgtttcg gttctgctgt tcataaatca 480
aatgcagctg ataacaaatc caaactccaa ggattcaaaa gtttagagac tacttcttcc 540
ttccaaatgg aaacaagttc tataaaagaa ggaaaagcaa aagtagggac tgcagatttt 600
cttattgagg ttgagaaaca aagagccata aaggtttttg ggaaaagcac aataattgga 660
ctggcgataa cattctttgc cgttccaaaa ctcaatgtgt acacagcctt cttctacttc 720
tccatctctt catttggggt tggtctgata ctaaacatca tatttctcta ttggcctata 780
cttggtctcc caagatcatc tttcaaggct tacctaaatg actggaacgg tcgaggctgg 840
tctttcttgg ctggatttct ctgtggattt ggtaatggtc ttcagtttat gggcggtcaa 900
gccgctggtt atgctgctgc aggagctgtt cagattgaaa ataaacattt tgggggatac 960
tgctgtttgg agaatacaaa agatcatcaa gaaaaacata tacacttctt atcagtatgc 1020
tatttatgtt catag 1035

<210> 4
<211> 1171

<212> DNA

<213> Arabidopsis thaliana

<220>

<223> UPS4-Gen

<400> 4

atgtatgata ctctcattgt gttgtttggg aagttggcca gcgattctga ctctattgga 60

gagaagaggt cgtcttcctc aacacacgtt tcttgatttc gcaaccgcga atctcttggc 120

tgcaatcgtg atagcatttt cgctcggtga gattgggaaa agcacgtttc taaaacctga 180

ctttaccact cagctacctc aggataattg gccgtcggta ttgcttgcag tcgcaggcgg 240

tgtgcttctt agcatcggga atttagcgac tcaatacgct tttgcgtttg tcggtttatc 300

ggttactgaa gttataaccg caagcattac cgtcgtcatt ggtacaacat tgaattattt 360

cttggacaac aagataaaca aagccgagat cctcttccct ggcgtaggtt gcttcttgat 420

cgctgttttc ctaggcgctg cggttcatgc ttctaatgca gctgatgtca aagagaaact 480

caagtcttta cctagcgaag atttatattc ttctattgaa aatggtgaag acaagcctga 540

aatagagaaa actgatgtcg aatctcaaga gaaactcgcg gaaaaggcga aagcagggac 600

ggcaggattc tatgtagagc tagagaacaa aagagcaatc aaggtctttg gaaaaagcat 660

aatgatcgga ctattcataa cgctgttcgc gggaatcagc ctttctctat tctctcctgc 720

attcaacttg gcaacaaacg atcaatggag tacattgcca aaaggcgtac ctaagctcgt 780

tgtctatacc gcctttttct acttctcaat cgcgggtttt ctcatttctt tgattctaaa 840

cctcatcttt ctttaccggc caatggtagg cttagcgaga tcatcgctta agaagtatat 900

atatgactcc aaaggtagag gttgggcggt tttcgctgga ttcttgtgcg ggtttggtaa 960

cggtttgcag ttcatgggag gtcaagccgc tggatacgcg gctgcagact ctgtccaggc 1020

acttcctctt gtaagcacat tttggggtat agttttgttc ggagagtatc gaaaatcgtc 1080

gaagcgaact tatgcccttt tagttagcat gttggccatg tttgtggcgg ccgtagctat 1140

tcttatggcc tcttccggtc accggaaatg a 1171

<210> 5

<211> 1224

<212> DNA

<213> Phaseolus vulgaris

<220>

<223> PvUPS-Gen

<400> 5
atgtatttgg tggagagcaa gggaggtgcc ataggatgca tgttcttggc tcttttcttc 60
ttgggaacat ggcctgccct tttgaccatg ttagagaggc gtggtcgtct tcctcagcat 120
acctaccttg attactcaat caccaatttc tttgctgctt tactcattgc ctttacattt 180
ggtgagatag gcaagggtaa acctgatgag ccaaatttct tagctcaact tgctcgggat 240
aattggccct cagttctttt tgcaatgggg ggtggtgtgg tccttagcct tgggaatcta 300
tcctcacaat atgcatttgc ttttgttggg ctatcggtta ctgaagtgat cacggcaagc 360
ataactgttg ttataggcac aaccgtgaat tactttttgg atgacaaaat caacaaagct 420
gagatccttt tcccaggagt tggttgcttt ttgattgcag tttttctagg ttctgccgtt 480
cattcatcca atgcttctga taataaagca aagctcaaca attactcaaa tgattataaa 540
gaagcagcca tctcttcaaa ggaaagagat ttagtgaaat caaaagatct ggagagggga 600
agtagttctg cagacaatgt tgaagcagga actgcagttt tcctcataga gcttgaggaa 660
agaagagcaa ttaaggtgtt tgggaaaagc actttgattg gattgtctct aactttctct 720
gctggacttt gcttctctat gttctcacca gcattcaact tggcaacaaa tgatcagtgg 780
cataccttgg aaaaagggat tccccatttg acagtttaca ctgccttctt ctatttttca 840
atctcttgtt ttgttattgc cataattcta aacatcacct tcctctacca ccctgtccta 900
aacctaccca agtcatcatt gaaggcttat ttggcagact ctgatggcag aatgtgggcc 960
ttgttggctg gtctcctttg tgggtttggg aatggtctcc aatttatggg gggtcaagct 1020
gcaggatatg cagcagcaga tgctgttcag gcacttccac ttgtaagcac tttctggggt 1080
gttgttctat ttggagagta cagaagatca tcaagaagaa catatgcact gctaggaagc 1140
atgttgttta tgtttattgt ggctgttggt gtgctgatgg catcatcagg gcacagaagc 1200
agttctcaca ctgccaagga ataa 1224

<210> 6
<211> 1134
<212> DNA
<213> Arabidopsis thaliana

<220>
<223> UPS5A-Gen

<400> 6
atgatagctc aagaattggg aatatatgtt gtggagagca agggaggagc aatattatgt 60
ctattgcttt ctctgctctg tttaggcact tggcctgctc ttatggctct tcttgaacgg 120

cgtggtcgtc tcccacaaca tacttatctt gattattcca tcactaactt tttggctgca 180
atctttatag cctttgtgtt tggtgggata ggtgaaagta cacatgaggc accaagtttc 240
attacacaac tcactcagat tcaagataat tggccctctg tgttgtttgc aatggcgggc 300
actactgtca attacttttt ggacaacgga ttgaacagag ctgatattct tttctctggt 360
gttggttgct tcatggtagc tgtttgtctt ggttctgctg ttcactcttc caattctgct 420
gatatcaagg cgaaactcgg aaaactctca ggggattgtg aaactgtgac ccctgaagag 480
tgccagagac tatttggagt agaagaagaa gaagaagaag aaaaagagat ggagaatgta 540
aaggaagggt ctgcagcttt tcttatagca ctcgaaaaca aaagagcaat caaagtgtta 600
ggaaagagca tggttgttgg tctgggaata actttttttg caggactttc tttctcattg 660
ttctcaccac tattcaacct tgcaacaaac gatcaatggc acactctaaa acaaggcgtt 720
cccaagctga ttgtctacac tgcgttcttc tacttctcac tatcttgctt tgtaatcgcg 780
gtcgctctca acattagttt cctgtacaaa cctgtgctag actcaccaag atcttccttt 840
agagaatacc tgagtgactg gaacggaaga ggttgggcac ttgcagctgg attgttatgt 900
gggtttggta acggtcttca gttcatgggt ggacaagctg caggatatgc agcctcagat 960
gctgttcagg cacttccttt agtatcgact tttgggggga tatatctgtt tggggagtat 1020
aggagatcat ctacgcgaac atatgctttg cttgttggaa tgctggtgat gtttactgtt 1080
gctgtggggc ttctaatggc ctcggctggg gaaagagaga cgcgatttac ttag 1134

<210> 7
<211> 1239
<212> DNA
<213> Arabidopsis thaliana

<220>
<223> UPS5B-Gen

<400> 7
atgatagctc aagaattggg aatatatgtt gtggagagca aaggaggagc aatattatgt 60
ctattgcttt ctctgctctg tttaggcact tggcctgctc ttatggctct tcttgaacgg 120
cgtggtcgtc tcccacaaca tacttatctt gattattcca tcactaactt tttggctgca 180
atctttatag cctttgtgtt tggtgggata ggtgaaagta cacatgaggc accaagtttc 240
attacacaac tcactcagat tcaagataat tggccctctg tgttgtttgc aatggcgggt 300
ggggttggtt taagtattgg aaaccttgct actcagtatt ctttagcttt tgttggtctg 360
tctgttacag aagtgacagc tgcgagcatc accgttgttg taggcactac tgtcaattac 420
tttttggaca acggattgaa cagagctgat attcttttct ctggtgttgg ttgcttcatg 480

gtagctgttt gtcttggttc tgctgttcac tcttccaatt ctgctgatat caaggcgaaa 540
ctcggaaaac tctcagggga ttgtgaaact gtgacccctg aagagtgcca gagactattt 600
ggagtagaag aagaagaaga agaagaaaaa gagatggaga atgtaaagga agggtctgcg 660
gcttttctta tagcactcga aaacaaaaga gcaatcaaag tgttaggaaa gagcatggtt 720
gttggtctgg gaataacttt ttttgcagga ctttctttct cattgttctc accactattc 780
aaccttgcaa caaacgatca atggcacact ctaaaacaag gcgttcccaa gctgattgtc 840
tacactgcgt tcttctactt ctcactatct tgctttgtaa tcgcggtcgc tctcaacatt 900
agtttcctgt acaaacctgt gctagactca ccaagatctt cctttagaga atacctgagt 960
gactggaacg gaagaggttg ggcacttgca gctggattgt tatgtgggtt tggtaacggt 1020
cttcagttca tgggtggaca agctgcagga tatgcagcct cagatgctgt tcaggcactt 1080
cctttagtat cgactttttg ggggatgtat ctgtttgggg agtataggag atcatctacg 1140
cgaacatatg ctttgcttgt tggaatgctg gtgatgttta ctgttgctgt ggggcttcta 1200
atggcctcgg ctggggaaag agagacgcga tttacttag 1239

<210> 8
<211> 390
<212> PRT
<213> Arabidopsis thaliana

<220>
<223> UPS1

<400> 8
Met Tyr Met Ile Glu Ser Lys Gly Gly Ala Ile Ala Cys Met Leu Leu
1               5                   10                  15

Ala Leu Leu Phe Leu Gly Thr Trp Pro Ala Ile Met Thr Leu Thr Glu
            20                  25                  30

Arg Arg Gly Arg Leu Pro Gln His Thr Tyr Leu Asp Tyr Thr Leu Thr
        35                  40                  45

Asn Leu Leu Ala Ala Val Ile Ile Ala Leu Thr Leu Gly Glu Ile Gly
    50                  55                  60

Pro Ser Arg Pro Asn Phe Phe Thr Gln Leu Ser Gln Asp Asn Trp Gln
65                  70                  75                  80

Ser Val Met Phe Ala Met Ala Gly Gly Ile Val Leu Ser Leu Gly Asn
                85                  90                  95

Leu Ala Thr Gln Tyr Ala Trp Ala Tyr Val Gly Leu Ser Val Thr Glu
            100                 105                 110

Val Ile Thr Ala Ser Ile Thr Val Val Ile Gly Thr Thr Leu Asn Tyr
        115                 120                 125

Phe Leu Asp Asp Arg Ile Asn Arg Ala Glu Val Leu Phe Pro Gly Val
    130                 135                 140

Ala Cys Phe Leu Ile Ala Val Cys Phe Gly Ser Ala Val His Lys Ser
145                 150                 155                 160

Asn Ala Ala Asp Asn Lys Thr Lys Leu Gln Asn Phe Lys Ser Leu Glu
                165                 170                 175

Thr Thr Ser Ser Phe Glu Met Glu Thr Ile Ser Ala Ser Asn Gly Leu
            180                 185                 190

Thr Lys Gly Lys Ala Lys Glu Gly Thr Ala Ala Phe Leu Ile Glu Leu
        195                 200                 205

Glu Lys Gln Arg Ala Ile Lys Val Phe Gly Lys Ser Thr Ile Ile Gly
    210                 215                 220

Leu Val Ile Thr Phe Phe Ala Gly Ile Cys Phe Ser Leu Phe Ser Pro
225                 230                 235                 240

Ala Phe Asn Leu Ala Thr Asn Asp Gln Trp His Thr Leu Lys His Gly
                245                 250                 255

Val Pro Lys Leu Asn Val Tyr Thr Ala Phe Phe Tyr Phe Ser Ile Ser
            260                 265                 270

Ala Phe Val Val Ala Leu Ile Leu Asn Ile Arg Phe Leu Tyr Trp Pro
        275                 280                 285

Ile Leu Gly Leu Pro Arg Ser Ser Phe Lys Ala Tyr Leu Asn Asp Trp
    290                 295                 300

Asn Gly Arg Gly Trp Ser Phe Leu Ala Gly Phe Leu Cys Gly Phe Gly
305                 310                 315                 320

Asn Gly Leu Gln Phe Met Gly Gly Gln Ala Ala Gly Tyr Ala Ala Ala
                325                 330                 335

Asp Ala Val Gln Ala Leu Pro Leu Val Ser Thr Phe Trp Gly Ile Leu
            340                 345                 350

Leu Phe Gly Glu Tyr Arg Arg Ser Ser Arg Lys Thr Tyr Thr Leu Leu
        355                 360                 365

Ile Ser Met Leu Leu Met Phe Ile Val Ala Val Ala Val Leu Met Ala
    370                 375                 380

Ser Ser Gly His Arg Lys
385                 390

<210> 9
<211> 398
<212> PRT
<213> Arabidopsis thaliana

<220>
<223> UPS2

<400> 9

Met Tyr Leu Val Glu Ser Lys Gly Gly Ala Ile Ala Cys Met Leu Leu
1               5                   10                  15

Ala Leu Leu Ser Leu Gly Thr Trp Pro Ala Val Leu Thr Leu Leu Glu
            20                  25                  30

Arg Arg Gly Arg Leu Pro Gln His Thr Tyr Leu Asp Tyr Ser Ile Thr
        35                  40                  45

Asn Leu Leu Ala Ala Ile Ile Ile Ala Phe Thr Phe Gly Gln Ile Gly
    50                  55                  60

Ser Thr Lys Pro Asp Ser Pro Asn Phe Ile Thr Gln Leu Ala Gln Asp
65                  70                  75                  80

Asn Trp Pro Ser Val Met Phe Ala Met Ala Gly Gly Ile Val Leu Ser
                85                  90                  95

Leu Gly Asn Leu Ser Thr Gln Tyr Ala Trp Ala Leu Val Gly Leu Ser
            100                 105                 110

Val Thr Glu Val Ile Thr Ser Ser Ile Thr Val Val Ile Gly Ser Thr
        115                 120                 125

Leu Asn Tyr Phe Leu Asp Asp Lys Ile Asn Lys Ala Glu Ile Leu Phe
    130                 135                 140

Pro Gly Val Ala Cys Phe Leu Ile Ala Val Cys Leu Gly Ser Ala Val
145                 150                 155                 160

His Arg Ser Asn Ala Asp Asp Asn Lys Ala Lys Leu Arg Asp Phe Glu
                165                 170                 175

Thr Ala Lys Gln Glu Ala Ser Gly Pro Ser Thr Glu Ile Gly Thr Asn
            180                 185                 190

Ser Ser Lys Asp Leu Glu Thr Asn Val Thr Thr Lys Pro Lys Glu Gly
        195                 200                 205

Thr Ala Arg Phe Leu Ile Glu Leu Glu Asn Thr Arg Ala Ile Lys Val
    210                 215                 220

Phe Gly Lys Arg Lys Ile Ile Gly Leu Ala Ile Thr Phe Phe Ala Gly
225                 230                 235                 240

Leu Cys Phe Ser Leu Phe Ser Pro Ala Phe Asn Leu Ala Thr Asn Asp
                245                 250                 255

Gln Trp Asn Arg Leu Lys Gln Gly Val Pro Lys Leu Val Val Tyr Thr
            260                 265                 270

Ala Phe Phe Tyr Phe Ser Val Ser Cys Phe Ile Ile Ala Leu Ile Leu
        275                 280                 285

Asn Val Val Phe Leu Tyr Tyr Pro Val Leu Gly Leu Pro Lys Ser Ser
    290                 295                 300

Phe Lys Ala Tyr Leu Asn Asp Trp Asn Gly Arg Tyr Trp Ala Phe Leu
305                 310                 315                 320

Ala Gly Phe Leu Cys Gly Phe Gly Asn Gly Leu Gln Phe Met Gly Gly
                325                 330                 335

Gln Ala Ala Gly Tyr Ala Ala Ala Asp Ser Val Gln Ala Leu Pro Leu
            340                 345                 350

Val Ser Thr Phe Trp Gly Val Val Leu Phe Gly Glu Tyr Arg Arg Ser
        355                 360                 365

Ser Arg Lys Thr Tyr Leu Leu Leu Phe Cys Met Leu Phe Met Phe Ile
    370                 375                 380

Ser Ala Val Ala Val Leu Met Ala Ser Ser Gly His Arg Lys
385                 390                 395

<210> 10
<211> 344
<212> PRT
<213> Arabidopsis thaliana

<220>
<223> UPS3

<400> 10
Met Tyr Val Ile Glu Ser Lys Gly Gly Thr Ile Thr Cys Met Leu Leu
1               5                   10                  15

Ala Leu Leu Phe Leu Gly Thr Trp Pro Ala Ile Met Thr Leu Thr Glu
            20                  25                  30

Arg Arg Gly Arg Leu Pro Gln His Thr Tyr Leu Asp Tyr Thr Leu Thr
        35                  40                  45

Asn Leu Leu Ala Ala Val Ile Ile Ala Phe Thr Leu Gly Glu Ile Ser
    50                  55                  60

Pro Ser Arg Pro Asn Phe Thr Thr Gln Leu Ser Gln Asp Asn Trp Pro
65                  70                  75                  80

Ser Val Met Phe Ala Met Ala Gly Gly Ile Phe Leu Ser Leu Gly Thr
                85                  90                  95

Leu Ala Thr Gln Tyr Ala Trp Ala Phe Val Gly Leu Ser Val Thr Glu
            100                 105                 110

Val Ile Thr Ala Ser Ile Ala Val Val Ile Gly Thr Thr Leu Asn Tyr

115                 120                 125

Phe Leu Asp Asp Arg Ile Asn Arg Ala Glu Val Leu Phe Pro Gly Val
    130                 135                 140

Ala Cys Phe Leu Ile Ala Val Cys Phe Gly Ser Ala Val His Lys Ser
145                 150                 155                 160

Asn Ala Ala Asp Asn Lys Ser Lys Leu Gln Gly Phe Lys Ser Leu Glu
                165                 170                 175

Thr Thr Ser Ser Phe Gln Met Glu Thr Ser Ser Ile Lys Glu Gly Lys
            180                 185                 190

Ala Lys Val Gly Thr Ala Asp Phe Leu Ile Glu Val Glu Lys Gln Arg
        195                 200                 205

Ala Ile Lys Val Phe Gly Lys Ser Thr Ile Ile Gly Leu Ala Ile Thr
    210                 215                 220

Phe Phe Ala Val Pro Lys Leu Asn Val Tyr Thr Ala Phe Phe Tyr Phe
225                 230                 235                 240

Ser Ile Ser Ser Phe Gly Val Gly Leu Ile Leu Asn Ile Ile Phe Leu
                245                 250                 255

Tyr Trp Pro Ile Leu Gly Leu Pro Arg Ser Ser Phe Lys Ala Tyr Leu
            260                 265                 270

Asn Asp Trp Asn Gly Arg Gly Trp Ser Phe Leu Ala Gly Phe Leu Cys
        275                 280                 285

Gly Phe Gly Asn Gly Leu Gln Phe Met Gly Gly Gln Ala Ala Gly Tyr
    290                 295                 300

Ala Ala Ala Gly Ala Val Gln Ile Glu Asn Lys His Phe Gly Gly Tyr

305                 310                 315                 320

Cys Cys Leu Glu Asn Thr Lys Asp His Gln Glu Lys His Ile His Phe
                325                 330                 335

Leu Ser Val Cys Tyr Leu Cys Ser
            340

<210> 11
<211> 401
<212> PRT
<213> Arabidopsis thaliana

<220>
<223> UPS4

<400> 11
Met Tyr Val Val Glu Ser Lys Ala Gly Ala Ile Gly Cys Met Ile Leu
  1               5                  10                  15

Ser Leu Cys Cys Leu Gly Ser Trp Pro Ala Ile Leu Thr Leu Leu Glu
             20                  25                  30

Arg Arg Gly Arg Leu Pro Gln His Thr Phe Leu Asp Phe Ala Thr Ala
         35                  40                  45

Asn Leu Leu Ala Ala Ile Val Ile Ala Phe Ser Leu Gly Glu Ile Gly
     50                  55                  60

Lys Ser Thr Phe Leu Lys Pro Asp Phe Thr Thr Gln Leu Pro Gln Asp
 65                  70                  75                  80

Asn Trp Pro Ser Val Leu Leu Ala Val Ala Gly Gly Val Leu Leu Ser
                 85                  90                  95

Ile Gly Asn Leu Ala Thr Gln Tyr Ala Phe Ala Phe Val Gly Leu Ser
            100                 105                 110

Val Thr Glu Val Ile Thr Ala Ser Ile Thr Val Val Ile Gly Thr Thr
        115                 120                 125

Leu Asn Tyr Phe Leu Asp Asn Lys Ile Asn Lys Ala Glu Ile Leu Phe
    130                 135                 140

Pro Gly Val Gly Cys Phe Leu Ile Ala Val Phe Leu Gly Ala Ala Val
145                 150                 155                 160

His Ala Ser Asn Ala Ala Asp Val Lys Glu Lys Leu Lys Ser Leu Pro
                165                 170                 175

Ser Glu Asp Leu Tyr Ser Ser Ile Glu Asn Gly Glu Asp Lys Pro Glu
            180                 185                 190

Ile Glu Lys Thr Asp Val Glu Ser Gln Glu Lys Leu Ala Glu Lys Ala
        195                 200                 205

Lys Ala Gly Thr Ala Gly Phe Tyr Val Glu Leu Glu Asn Lys Arg Ala
    210                 215                 220

Ile Lys Val Phe Gly Lys Ser Ile Met Ile Gly Leu Phe Ile Thr Leu
225                 230                 235                 240

Phe Ala Gly Ile Ser Leu Ser Leu Phe Ser Pro Ala Phe Asn Leu Ala
                245                 250                 255

Thr Asn Asp Gln Trp Ser Thr Leu Pro Lys Gly Val Pro Lys Leu Val
            260                 265                 270

Val Tyr Thr Ala Phe Phe Tyr Phe Ser Ile Ala Gly Phe Leu Ile Ser
        275                 280                 285

Leu Ile Leu Asn Leu Ile Phe Leu Tyr Arg Pro Met Val Gly Leu Ala
    290                 295                 300

Arg Ser Ser Leu Lys Lys Tyr Ile Tyr Asp Ser Lys Gly Arg Gly Trp
305                 310                 315                 320

Ala Val Phe Ala Gly Phe Leu Cys Gly Phe Gly Asn Gly Leu Gln Phe
                325                 330                 335

Met Gly Gly Gln Ala Ala Gly Tyr Ala Ala Ala Asp Ser Val Gln Ala
            340                 345                 350

Leu Pro Leu Val Ser Thr Phe Trp Gly Ile Val Leu Phe Gly Glu Tyr
        355                 360                 365

Arg Lys Ser Ser Lys Arg Thr Tyr Ala Leu Leu Val Ser Met Leu Ala
    370                 375                 380

Met Phe Val Ala Ala Val Ala Ile Leu Met Ala Ser Ser Gly His Arg
385                 390                 395                 400

Lys

<210> 12
<211> 407
<212> PRT
<213> Phaseolus vulgaris

<220>
<223> PvUPS

<400> 12
Met Tyr Leu Val Glu Ser Lys Gly Gly Ala Ile Gly Cys Met Phe Leu
  1               5                  10                  15

Ala Leu Phe Phe Leu Gly Thr Trp Pro Ala Leu Leu Thr Met Leu Glu
            20                  25                  30

Arg Arg Gly Arg Leu Pro Gln His Thr Tyr Leu Asp Tyr Ser Ile Thr
        35                  40                  45

Asn Phe Phe Ala Ala Leu Leu Ile Ala Phe Thr Phe Gly Glu Ile Gly
    50                  55                  60

Lys Gly Lys Pro Asp Glu Pro Asn Phe Leu Ala Gln Leu Ala Arg Asp
65                  70                  75                  80

Asn Trp Pro Ser Val Leu Phe Ala Met Gly Gly Gly Val Val Leu Ser
                85                  90                  95

Leu Gly Asn Leu Ser Ser Gln Tyr Ala Phe Ala Phe Val Gly Leu Ser
            100                 105                 110

Val Thr Glu Val Ile Thr Ala Ser Ile Thr Val Val Ile Gly Thr Thr
        115                 120                 125

Val Asn Tyr Phe Leu Asp Asp Lys Ile Asn Lys Ala Glu Ile Leu Phe
    130                 135                 140

Pro Gly Val Gly Cys Phe Leu Ile Ala Val Phe Leu Gly Ser Ala Val
145                 150                 155                 160

His Ser Ser Asn Ala Ser Asp Asn Lys Ala Lys Leu Asn Asn Tyr Ser
                165                 170                 175

Asn Asp Tyr Lys Glu Ala Ala Ile Ser Ser Lys Glu Arg Asp Leu Val
            180                 185                 190

Lys Ser Lys Asp Leu Glu Arg Gly Ser Ser Ser Ala Asp Asn Val Glu
        195                 200                 205

Ala Gly Thr Ala Val Phe Leu Ile Glu Leu Glu Glu Arg Arg Ala Ile
    210                 215                 220

Lys Val Phe Gly Lys Ser Thr Leu Ile Gly Leu Ser Leu Thr Phe Ser
225                 230                 235                 240

Ala Gly Leu Cys Phe Ser Met Phe Ser Pro Ala Phe Asn Leu Ala Thr
                245                 250                 255

Asn Asp Gln Trp His Thr Leu Glu Lys Gly Ile Pro His Leu Thr Val
            260                 265                 270

Tyr Thr Ala Phe Phe Tyr Phe Ser Ile Ser Cys Phe Val Ile Ala Ile
        275                 280                 285

Ile Leu Asn Ile Thr Phe Leu Tyr His Pro Val Leu Asn Leu Pro Lys
    290                 295                 300

Ser Ser Leu Lys Ala Tyr Leu Ala Asp Ser Asp Gly Arg Met Trp Ala
305                 310                 315                 320

Leu Leu Ala Gly Leu Leu Cys Gly Phe Gly Asn Gly Leu Gln Phe Met
                325                 330                 335

Gly Gly Gln Ala Ala Gly Tyr Ala Ala Ala Asp Ala Val Gln Ala Leu
            340                 345                 350

Pro Leu Val Ser Thr Phe Trp Gly Val Val Leu Phe Gly Glu Tyr Arg
        355                 360                 365

Arg Ser Ser Arg Arg Thr Tyr Ala Leu Leu Gly Ser Met Leu Phe Met
    370                 375                 380

Phe Ile Val Ala Val Gly Val Leu Met Ala Ser Ser Gly His Arg Ser
385                 390                 395                 400

Ser Ser His Thr Ala Lys Glu
                405

<210> 13
<211> 377
<212> PRT
<213> Arabidopsis thaliana

<220>
<223> UPS5A

<400> 13
Met Ile Ala Gln Glu Leu Gly Ile Tyr Val Val Glu Ser Lys Gly Gly
1               5                   10                  15

Ala Ile Leu Cys Leu Leu Leu Ser Leu Leu Cys Leu Gly Thr Trp Pro
            20                  25                  30

Ala Leu Met Ala Leu Leu Glu Arg Arg Gly Arg Leu Pro Gln His Thr
        35                  40                  45

Tyr Leu Asp Tyr Ser Ile Thr Asn Phe Leu Ala Ala Ile Phe Ile Ala
    50                  55                  60

Phe Val Phe Gly Gly Ile Gly Glu Ser Thr His Glu Ala Pro Ser Phe
65                  70                  75                  80

Ile Thr Gln Leu Thr Gln Ile Gln Asp Asn Trp Pro Ser Val Leu Phe
                85                  90                  95

Ala Met Ala Gly Thr Thr Val Asn Tyr Phe Leu Asp Asn Gly Leu Asn
            100                 105                 110

Arg Ala Asp Ile Leu Phe Ser Gly Val Gly Cys Phe Met Val Ala Val

        115                 120                 125

Cys Leu Gly Ser Ala Val His Ser Ser Asn Ser Ala Asp Ile Lys Ala
    130                 135                 140

Lys Leu Gly Lys Leu Ser Gly Asp Cys Glu Thr Val Thr Pro Glu Glu
145                 150                 155                 160

Cys Gln Arg Leu Phe Gly Val Glu Glu Glu Glu Glu Glu Glu Lys Glu
                165                 170                 175

Met Glu Asn Val Lys Glu Gly Ser Ala Ala Phe Leu Ile Ala Leu Glu
            180                 185                 190

Asn Lys Arg Ala Ile Lys Val Leu Gly Lys Ser Met Val Val Gly Leu
        195                 200                 205

Gly Ile Thr Phe Phe Ala Gly Leu Ser Phe Ser Leu Phe Ser Pro Leu
    210                 215                 220

Phe Asn Leu Ala Thr Asn Asp Gln Trp His Thr Leu Lys Gln Gly Val
225                 230                 235                 240

Pro Lys Leu Ile Val Tyr Thr Ala Phe Phe Tyr Phe Ser Leu Ser Cys
                245                 250                 255

Phe Val Ile Ala Val Ala Leu Asn Ile Ser Phe Leu Tyr Lys Pro Val
            260                 265                 270

Leu Asp Ser Pro Arg Ser Ser Phe Arg Glu Tyr Leu Ser Asp Trp Asn
        275                 280                 285

Gly Arg Gly Trp Ala Leu Ala Ala Gly Leu Leu Cys Gly Phe Gly Asn
    290                 295                 300

Gly Leu Gln Phe Met Gly Gly Gln Ala Ala Gly Tyr Ala Ala Ser Asp

305                 310                 315                 320

Ala Val Gln Ala Leu Pro Leu Val Ser Thr Phe Trp Gly Ile Tyr Leu
                325                 330                 335

Phe Gly Glu Tyr Arg Arg Ser Ser Thr Arg Thr Tyr Ala Leu Leu Val
            340                 345                 350

Gly Met Leu Val Met Phe Thr Val Ala Val Gly Leu Leu Met Ala Ser
        355                 360                 365

Ala Gly Glu Arg Glu Thr Arg Phe Thr
    370                 375

<210> 14
<211> 412
<212> PRT
<213> Arabidopsis thaliana

<220>
<223> UPS5B

<400> 14
Met Ile Ala Gln Glu Leu Gly Ile Tyr Val Val Glu Ser Lys Gly Gly
 1               5                  10                  15

Ala Ile Leu Cys Leu Leu Leu Ser Leu Leu Cys Leu Gly Thr Trp Pro
            20                  25                  30

Ala Leu Met Ala Leu Leu Glu Arg Arg Gly Arg Leu Pro Gln His Thr
        35                  40                  45

Tyr Leu Asp Tyr Ser Ile Thr Asn Phe Leu Ala Ala Ile Phe Ile Ala
    50                  55                  60

Phe Val Phe Gly Gly Ile Gly Glu Ser Thr His Glu Ala Pro Ser Phe
65                  70                  75                  80

Ile Thr Gln Leu Thr Gln Ile Gln Asp Asn Trp Pro Ser Val Leu Phe
                85                  90                  95

Ala Met Ala Gly Gly Val Gly Leu Ser Ile Gly Asn Leu Ala Thr Gln
            100                 105                 110

Tyr Ser Leu Ala Phe Val Gly Leu Ser Val Thr Glu Val Thr Ala Ala
        115                 120                 125

Ser Ile Thr Val Val Val Gly Thr Thr Val Asn Tyr Phe Leu Asp Asn
    130                 135                 140

Gly Leu Asn Arg Ala Asp Ile Leu Phe Ser Gly Val Gly Cys Phe Met
145                 150                 155                 160

Val Ala Val Cys Leu Gly Ser Ala Val His Ser Ser Asn Ser Ala Asp
                165                 170                 175

Ile Lys Ala Lys Leu Gly Lys Leu Ser Gly Asp Cys Glu Thr Val Thr
            180                 185                 190

Pro Glu Glu Cys Gln Arg Leu Phe Gly Val Glu Glu Glu Glu Glu Glu
        195                 200                 205

Glu Lys Glu Met Glu Asn Val Lys Glu Gly Ser Ala Ala Phe Leu Ile
    210                 215                 220

Ala Leu Glu Asn Lys Arg Ala Ile Lys Val Leu Gly Lys Ser Met Val
225                 230                 235                 240

Val Gly Leu Gly Ile Thr Phe Phe Ala Gly Leu Ser Phe Ser Leu Phe
                245                 250                 255

Ser Pro Leu Phe Asn Leu Ala Thr Asn Asp Gln Trp His Thr Leu Lys
            260                 265                 270

Gln Gly Val Pro Lys Leu Ile Val Tyr Thr Ala Phe Phe Tyr Phe Ser
        275                 280                 285

Leu Ser Cys Phe Val Ile Ala Val Ala Leu Asn Ile Ser Phe Leu Tyr
    290                 295                 300

Lys Pro Val Leu Asp Ser Pro Arg Ser Ser Phe Arg Glu Tyr Leu Ser
305                 310                 315                 320

Asp Trp Asn Gly Arg Gly Trp Ala Leu Ala Ala Gly Leu Leu Cys Gly
                325                 330                 335

Phe Gly Asn Gly Leu Gln Phe Met Gly Gly Gln Ala Ala Gly Tyr Ala
            340                 345                 350

Ala Ser Asp Ala Val Gln Ala Leu Pro Leu Val Ser Thr Phe Trp Gly
        355                 360                 365

Met Tyr Leu Phe Gly Glu Tyr Arg Arg Ser Ser Thr Arg Thr Tyr Ala
    370                 375                 380

Leu Leu Val Gly Met Leu Val Met Phe Thr Val Ala Val Gly Leu Leu
385                 390                 395                 400

Met Ala Ser Ala Gly Glu Arg Glu Thr Arg Phe Thr
                405                 410

<210> 15
<211> 17
<212> PRT
<213> Unbekannt

<220>
<223> Beschreibung des unbekannten
      Organismus:Consensus-Sequenz

<400> 15
Phe Leu Xaa Xaa Xaa Glu Xaa Xaa Arg Ala Ile Lys Val Leu Gly Lys
  1               5                  10                  15

Ser

<210> 16
<211> 17
<212> PRT
<213> Unbekannt

<220>
<223> Beschreibung des unbekannten
      Organismus:Consensus-Sequenz

<400> 16
Phe Leu Xaa Xaa Xaa Glu Xaa Xaa Arg Ala Ile Lys Val Leu Gly Lys
  1               5                  10                  15

Arg

<210> 17
<211> 17
<212> PRT
<213> Unbekannt

<220>
<223> Beschreibung des unbekannten

Organismus:Consus-Sequenz

<400> 17
Phe Leu Xaa Xaa Xaa Glu Xaa Xaa Arg Ala Ile Lys Val Phe Gly Lys
1               5                   10                  15

Ser

<210> 18
<211> 17
<212> PRT
<213> Unbekannt

<220>
<223> Beschreibung des unbekannten
      Organismus:Consus-Sequenz

<400> 18
Phe Leu Xaa Xaa Xaa Glu Xaa Xaa Arg Ala Ile Lys Val Phe Gly Lys
1               5                   10                  15

Arg

<210> 19
<211> 17
<212> PRT
<213> Unbekannt

<220>
<223> Beschreibung des unbekannten
      Organismus:Consus-Sequenz

<400> 19
Phe Tyr Xaa Xaa Xaa Glu Xaa Xaa Arg Ala Ile Lys Val Phe Gly Lys
1               5                   10                  15

Ser

<210> 20
<211> 17
<212> PRT
<213> Unbekannt

<220>
<223> Beschreibung des unbekannten
      Organismus:Consus-Sequenz

<400> 20
Phe Tyr Xaa Xaa Xaa Glu Xaa Xaa Arg Ala Ile Lys Val Phe Gly Lys
1               5                   10                  15

Arg

<210> 21
<211> 17
<212> PRT
<213> Unbekannt

<220>
<223> Beschreibung des unbekannten
      Organismus:Consus-Sequenz

<400> 21
Phe Tyr Xaa Xaa Xaa Glu Xaa Xaa Arg Ala Ile Lys Val Leu Gly Lys

1                   5                   10                  15

Ser

<210> 22
<211> 17
<212> PRT
<213> Unbekannt

<220>
<223> Beschreibung des unbekannten
      Organismus:Consus-Sequenz

<400> 22
Phe Tyr Xaa Xaa Xaa Glu Xaa Xaa Arg Ala Ile Lys Val Leu Gly Lys
  1               5                  10                  15

Arg

## Beschreibung

Die Erfindung betrifft Nukleinsäuren, die die codierende Region für ein Transportprotein für stickstoffhaltige Heterozyklen, insbesondere deren Oxoderivate, enthalten. Die Erfindung betrifft ferner die Verwendung der Nukleinsäuren sowie Vektoren, mobile Elemente, Wirtszellen und Pflanzen sowie deren Teile und Samen, die solche Nukleinsäuren enthalten. Weiter betrifft die Erfindung auch Verfahren zur Herstellung von Pflanzen, die mit den Nukleinsäuren transformiert sind. Darüber hinaus betrifft die Erfindung auch Transportproteine für stickstoffhaltige Heterozyklen.

Transporter spielen für lebende Organismen eine besondere Rolle. Sie entscheiden über die Aufnahme oder Abgabe eines Stoffes in eine oder aus einer Zelle bzw. einem Organismus und steuern darüber hinaus den Transport und die Verteilung der Stoffe zwischen Zellen. Transporter stehen in der Regel am Anfang oder Ende eines Stoffwechselwegs und nehmen daher grundsätzlich übergeordnete Kontrollfunktionen wahr. In höheren Pflanzen sind Transportprozesse zur Verteilung von Assimilaten, Metaboliten und Phytohormonen von hoher Bedeutung. In diesem Zusammenhang sind insbesondere Transportprozesse für stickstoffhaltige Heterozyklen zu erwähnen, da diese Verbindungen im Stoffwechsel der Pflanze in zentraler Rolle mitwirken.

So wird bei einigen tropischen Leguminosen, wie z.B. der wirtschaftlich sehr wichtigen Sojabohne, der in den Wurzelknöllchen fixierte Stickstoff in Form von Allantoin und seinem Abbauprodukt Allantoat aus den Wurzelknöllchen in den Sproß transportiert.

Auch in Nicht-Leguminosen spielen derartige Transporter aber wahrscheinlich eine wichtige Rolle, beispielsweise während der Seneszenz. In allen Pflanzen liegt z.B. mit den Nukleobasen eine beträchtliche Menge des Gesamtstickstoffs in Form von N-haltigen Heterozyklen vor. Während der Seneszenz kommt es zu einer allgemeinen Mobilisierung der Nährstoffe in den absterbenden Geweben, die dann in entsprechende Speicherorgane oder Früchte transportiert werden. Durch den Abtransport und den Abbau von N-haltigen Heterozyklen kann der in ihnen enthaltene Stickstoff wieder zurückgewonnen werden.

Darüber hinaus spielen N-haltige Heterozyklen, insbesondere deren Oxoderivate, auch im Sekundärstoffwechsel der Pflanze, beispielsweise bei der Signalübertragung durch Hormone, eine wichtige Rolle. In diesem Zusammenhang sind z.B. Verbindungen wie Nikotin, Barbitursäure oder Coffein zu nennen. Ferner sind Pestizide, die Derivate von Hydantoin darstellen, eine weitere wichtige Klasse von Oxoderivaten N-haltiger Heterozyklen.

Bei Pflanzen ist über die beteiligten Transportproteine trotz ihrer erheblichen Bedeutung bislang wenig bekannt. In DE 19907209, WO 00/49152 sowie in Gillissen et al. (2000), The Plant Cell 12, 291-300, ist eine Familie von Transportproteinen (AtPUP) aus *Arabidopsis* beschrieben, die beispielsweise Adenin und Cytosin transportieren. Nachteil dieser Transportproteine ist aber, daß sie wichtige Verbindungen wie z.B. Allantoin, Hydantoin, Oxypurinol, Uracil, Urat (Harnsäure oder Salze hiervon) oder Xanthin nicht transportieren können.

Dabei wäre es wünschenswert, wenn solche Proteine bzw. hierfür codierende Nukleinsäuren verfügbar wären, um bei Nutzpflanzen gezielt in den Stoffwechsel dieser Verbindungen und von Derivaten dieser Verbindungen eingreifen zu können.

Bislang ist man weitgehend auf rein züchterische Maßnahmen angewiesen, um beispielsweise bei Sojabohnen eine Verbesserung des Öl/Protein-Verhältnisses zu erzielen.

Aufgabe der vorliegenden Erfindung ist es daher, Nukleinsäuren zur Verfügung zu stellen, die eine gezielte Kontrolle des Im- und Exports von N-haltigen Heterozyklen, die von den bekannten Transportern nicht transportiert werden, bei Pflanzen ermöglichen.

Diese Aufgabe wird erfindungsgemäß gelöst durch eine Nukleinsäure, die die codierende Region für ein Transportprotein für stickstoffhaltige Heterozyklen, insbesondere deren Oxoderivate, enthält, wobei die Nukleinsäure für ein Transportprotein codiert, das eine oder mehrere Verbindungen, ausgewählt aus der Gruppe bestehend aus einem Oxoderivat mit mindestens zwei Oxo-Gruppen im Ringsystem des Heterozyklus, ausgenommen Koffein, und einem Oxoderivat mit mindestens drei Oxo-Gruppen im Ringsystem des Heterozyklus, transportiert. Bevorzugte Oxoderivate mit mindestens zwei Oxo-Gruppen (-CO-) im Ringsystem sind Hydantoin, Oxypurinol, Uracil und Xanthin. Bevorzugte Oxoderivate mit mindestens drei Oxo-Gruppen im Ringsystem sind Allantoin und Urat.

Die vorliegende Erfindung betrifft auch eine Nukleinsäure, die die codierende Region für ein Transportprotein für stickstoffhaltige Heterozyklen, insbesondere deren Oxoderivate, enthält, wobei die Nukleinsäure
a) eine DNA-Sequenz, die für ein Peptid gemäß einer der Sequenzen der SEQ ID NO: 15 bis SEQ ID NO: 22 codiert, oder
b) eine zu der DNA-Sequenz gemäß a) komplementäre Nukleotidsequenz, oder
c) eine der DNA-Sequenz gemäß a) entsprechende RNA-Sequenz oder DNA/RNA-Mischsequenz umfaßt, oder
d) mit einer Nukleinsäure, die die DNA-Sequenz gemäß a) oder eine zu der DNA-Sequenz gemäß a) komplementäre Nukleotidsequenz aufweist, hybridisiert.

Die genannten Sequenzen der SEQ ID NO: 15 bis SEQ ID NO: 22 sind Varianten der folgenden Consensus-Sequenz, die eine potentielle ATP-Bindungsstelle enthält und Transportproteinen der hier erstmals beschriebenen UPS-Familie gemeinsam ist:

Phe-[Leu,Tyr]-Xaa-Xaa-Xaa-Glu-Xaa-Xaa-Arg-Ala-Ile-Lys-
Val-[Leu,Phe]-Gly-Lys-[Ser,Arg]

Die Angabe von Aminosäuren in eckigen Klammern bedeutet hierbei, daß sowohl die eine als auch die andere der in der Klammer angegebenen Aminosäure an dieser Stelle in der Sequenz vorkommen kann.

Die erfindungsgemäßen Nukleinsäuren codieren für Transportproteine, die in verschiedenen Geweben vorkommen und sowohl den Im- als auch den Export von N-haltigen Heterozyklen, insbesondere von deren Oxoderivaten, kontrollieren.

Der hier verwendete Begriff "N-haltige Heterozyklen" umfaßt Nukleobasen und ihre Derivate sowie mit Nukleobasen chemisch verwandte Stoffe. Hierzu zählen beispielsweise die Purinbasen Adenin und Guanin sowie Derivate wie Xanthin, Hypoxanthin, Allantoin, Urat (Harnsäure und deren Salze), Xanthosin oder Inosin. Auch Cytokinine, wie z.B. Zeatin, Isopentenyladenin oder Kinetin und bestimmte Alkaloide, wie z.B. Coffein, Theophyllin, Theobromin oder Nikotin werden von diesem Begriff umfaßt. Zu den N-haltigen Heterozyklen zählen aber auch die Pyrimidinbasen Cytosin, Thymin und Uracil und Derivate wie beispielsweise Barbiturate. Auch Folsäure, Oxypurinol und Allopurinol werden von dem Begriff umfaßt. Weiterhin können die Nukleobasen auch modifiziert und beispielsweise an Zucker oder andere Bausteine gekoppelt sein. Somit umfaßt der Begriff auch Riboside wie z.B. Adenosin oder Cytidin, aber auch Cytokininriboside.

Der hier verwendete Begriff "Oxoderivate von N-haltigen Heterozyklen" bedeutet, daß wenigstens eine Oxo-Gruppe (-CO-) im Ringsystem des Heterozyklus vorkommt. Danach sind beispielsweise Allantoin, Allopurinol, Barbiturate, Cytosin, Guanin, Folsäure, Hydantoin, Inosin, Oxypurinol, Paraxanthin, Theobromin, Theophyllin, Thymin, Uracil, Urat, Xanthin und Xanthosin Oxoderivate von N-haltigen Heterozyklen.

Die erfindungsgemäßen Nukleinsäuren codieren für Proteine (im folgenden auch kurz als "N-Heterozyklentransporter" bezeichnet), die an dem Transport von N-haltigen Heterozyklen, also beispielsweise einer der vorgenannten Verbindungen, durch eine Biomembran beteiligt sind. Dieser Transport kann aktiv oder passiv erfolgen. Zum Nachweis der Transporteraktivität kann beispielsweise die von Ninnemann et al. (1994, EMBO J. 15, 3464-3471) beschriebene Methode angewendet werden. Die Transportproteine weisen keinerlei Verwandtschaft zu dem für Hefe (*Saccharomyces cerevisiae*) bekannten Allantoin-Transporter DAL4 auf und sind auch nicht mit der PUP-Familie (Gillissen et al., 2000, The Plant Cell 12, 291-300) verwandt.

Die Nukleotidsequenzen der Nukleinsäuren gemäß der vorliegenden Erfindung können DNA- oder RNA-Sequenzen, aber auch Mischsequenzen aus DNA- und RNA-Nukleotiden sein. Der Begriff "eine der DNA-Sequenz entsprechende RNA-Sequenz" bezeichnet hierbei eine RNA-Sequenz, die die gleiche Abfolge der Purin- und Pyrimidinbasen wie eine DNA-Sequenz, anstelle der Base Thymin in der DNA-Sequenz aber die Base Uracil aufweist. Unter einer DNA/RNA-Mischsequenz wird hier eine Nukleotidsequenz verstanden, die sowohl DNA-Nukleotide als auch RNA-Nukleotide enthält. Chimäre Oligonukleotide stellen beispielsweise solche Mischsequenzen dar.

Der hier verwendete Begriff "Hybridisieren" bedeutet Hybridisieren unter üblichen Bedingungen, wie sie in Sambrook et al. (Molecular Cloning. A laboratory manual, Cold Spring Harbor Laboratory Press, 2. Aufl., 1989) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

Bevorzugt umfaßt die erfindungsgemäße Nukleinsäure
a) eine DNA-Sequenz der SEQ ID NO: 1, SEQ ID NO: 2, SEQ ID NO: 3, SEQ ID NO: 4 oder SEQ ID NO: 5, SEQ ID NO: 6 oder SEQ ID NO: 7, oder
b) eine zu der DNA-Sequenz der SEQ ID NO: 1, SEQ ID NO: 2, SEQ ID NO: 3, SEQ ID NO: 4 oder SEQ ID NO: 5, SEQ ID NO: 6 oder SEQ ID NO: 7 komplementäre Nukleotidsequenz, oder
c) eine der DNA-Sequenz der SEQ ID NO: 1, SEQ ID NO: 2, SEQ ID NO: 3, SEQ ID NO: 4 oder SEQ ID NO: 5, SEQ ID NO: 6 oder SEQ ID NO: 7 entsprechende RNA-Sequenz oder DNA/RNA-Mischsequenz, oder
d) hybridisiert mit einer Nukleinsäure, die die DNA-Sequenz der SEQ ID NO: 1, SEQ ID NO: 2, SEQ ID NO: 3, SEQ ID NO: 4 oder SEQ ID NO: 5, SEQ ID NO: 6 oder SEQ ID NO: 7 oder eine zu der DNA-Sequenz der SEQ ID NO: 1, SEQ ID NO: 2, SEQ ID NO: 3, SEQ ID NO: 4 oder SEQ ID NO: 5, SEQ ID NO: 6 oder SEQ ID NO: 7 komplementäre Nukleotidsequenz aufweist.

Die Erfindung betrifft auch Nukleinsäurekonstrukte, Vektoren oder mobile genetische Elemente, die mindestens eine Nukleinsäure gemäß der vorliegenden Erfindung enthalten.

Nukleinsäurekonstrukte gemäß der Erfindung sind aus mehreren Nukleinsäuren zusammengesetzt und enthalten neben einer erfindungsgemäßen Nukleinsäure beispielsweise auch ein regulierendes Elementen, das die Expression der Nukleinsäure in einer Zelle kontrolliert. Beispiele für solche regulierenden Elemente sind konstitutive oder induzierbare Promotoren, wie z.B. der virale CaMV35S-Promotor (Pietrzak et al., 1986, Nucl. Acids Res. 14, 5857-5868). Das Konstrukt kann auch in einem Vektor oder mobilen genetischen Element vorliegen.

Vektoren oder mobile genetische Elemente, die zur Einführung von Nukleinsäuren in Wirtszellen geeignet sind, beispielsweise Viren, Bakteriophagen, Cosmide, Plasmide, künstliche Hefechromosomen, T-DNA, Transposons, Insertionssequenzen usw., sind dem Fachmann auf dem Gebiet molekularer Klonierungstechniken wohlbekannt.

Bevorzugt ist die erfindungsgemäße Nukleinsäure in dem Konstrukt, dem Vektor oder mobilen genetischen Element in Antisense-Orientierung enthalten. "Antisense-Orientierung" einer Nukleinsäure bedeutet in Bezug auf DNA, daß eine Transkription der Nukleinsäure in einer mRNA resultiert, deren Nukleotidsequenz komplementär ist zu der natürlichen (endogenen) mRNA, so daß deren Translation behindert oder verhindert wird. "Antisense-Orientierung" einer RNA bedeutet, daß die RNA-Sequenz zu einer endogenen mRNA komplementär ist und deren Translation durch Anlagerung behindert oder verhindert. Wird eine erfindungsgemäße Nukleinsäure in Antisense-Orientierung beispielsweise mit Hilfe eines Vektors in das Genom einer Pflanzenzelle eingeführt, kann dies zur Unterdrückung der Bildung der pflanzeneigenen N-Heterozyklentransporter-Moleküle führen. Hierbei ist die Translation der mRNA des endogenen N-Heterozyklentransporters durch Anlagerung einer Antisense-RNA an die mRNA behindert. Im Falle der Einführung einer DNA-Sequenz wird die Antisense-RNA durch Transkription der DNA-Sequenz gebildet. Im Falle einer der DNA-Sequenz entsprechenden RNA-Sequenz kann diese selbst die Antisense-RNA darstellen. Eine posttranskriptionelle Stummschaltung (PTGS = posttranscriptional gene silencing) des endogenen N-Heterozyklentransportergens kann auch durch Co-Suppression oder RNA-Interferenz herbeigeführt werden, indem die DNA-Sequenz oder eine der DNA-Sequenz entsprechende RNA-Sequenz in Sense-Orientierung in die Pflanzenzelle eingeführt wird, wobei die RNA im Falle der RNA-Interferenz als Doppelstrang-RNA verwendet wird. Darüber hinaus ist es auch möglich, aus RNA- und DNA-Sequenzen zusammengesetzte Nukleinsäuren, chimäre Oligonukleotide, wie sie beispielsweise von Rice et al., 2000, Plant Physiology 123, 427-437, beschrieben werden, einzusetzen, um ein Stummschalten des endogenen N-Heterozyklentransportergens zu bewirken. Die betroffenen Zellen sind weitgehend daran gehindert, N-Heterozyklentransporter zu bilden. Durch eine Kopplung an geeignete gewebespezifische Promotoren ist es auch möglich, gezielt die Bildung von N-Heterozyklentransportern in bestimmten Geweben oder Organen der Pflanze, etwa in den Blättern, zu vermindern oder zu unterdrücken.

Ferner betrifft die Erfindung eukaryotische oder prokaryotische Wirtszellen, die mindestens eine Nukleinsäure gemäß der vorliegenden Erfindung enthalten. Bevorzugt ist die Nukleinsäure hierbei in Antisense-Orientierung in der Wirtszelle enthalten. Sie kann aber auch in Sense-Orientierung in einem RNA-Doppelstrang oder einem chimären Oligonukleotid in der Wirtszelle enthalten sein. Dies ermöglicht ebenfalls die Stummschaltung des endogenen N-Heterozyklentransportergens.

Die Nukleinsäure können beispielsweise durch Transformation, Mikroinjektion, Protoplastenfusion, Elektroporation, Virusinfektion oder andere Techniken, die dem Fachmann bekannt sind, in Pflanzenzellen eingebracht werden.

Die Erfindung betrifft auch Pflanzen sowie Teile oder Samen von Pflanzen, die mit mindestens einer Nukleinsäure gemäß der vorliegenden Erfindung transformiert sind. Die Pflanzen können Leguminosen, beispielsweise Sojapflanzen, Erbsen, Bohnen oder Erdnüsse sein. Es kommen aber auch zahlreiche andere Pflanzen in Frage, beispielsweise Kartoffeln, Tomaten, Baumwolle, Zuckerrüben, Zuckerrohr, Tabak, Kaffee, Raps, *Ricinus*, Reis- oder Maispflanzen usw. Bevorzugt sind die transgenen Pflanzen, deren Teile oder deren Samen mit der Nukleinsäure in Antisense-Orientierung transformiert. Hierdurch sind beispielsweise Pflanzen erhältlich, bei denen die Expression eines N-Heterozyklentransportergens unterdrückt ist.

Die Erfindung betrifft auch Transportproteine für stickstoffhaltige Heterozyklen, insbesondere deren Oxoderivate, die eine oder mehrere Verbindungen, ausgewählt aus der Gruppe bestehend aus einem Oxoderivat mit mindestens zwei Oxo-Gruppen im Ringsystem des Heterozyklus, ausgenommen Koffein, und einem Oxoderivat mit mindestens drei Oxo-Gruppen im Ringsystem des Heterozyklus, transportieren. Bevorzugt handelt es sich bei dem Oxoderivat mit mindestens zwei Oxo-Gruppen im Ringsystem um Hydantoin, Oxypurinol, Uracil und Xanthin. Bevorzugte Oxoderivate mit mindestens drei Oxo-Gruppen im Ringsystem sind Allantoin und Urat.

Die Erfindung betrifft darüber hinaus Transportproteine für stickstoffhaltige Heterozyklen, insbesondere deren Oxoderivate, deren Aminosäuresequenz
a) die Aminosäuresequenz der SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22, oder
b) eine zu der Aminosäuresequenz der SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22 homologe Aminosäuresequenz umfaßt.

Die genannten Aminosäuresequenzen bilden die bereits oben beschriebene Consensus-Sequenz.

Bevorzug umfaßt die Aminosäuresequenz der erfindungsgemäßen Tranportproteine die Aminosäuresequenz der SEQ ID NO: 8, SEQ ID NO: 9, SEQ ID NO: 10, SEQ ID NO: 11, SEQ ID NO: 12, SEQ ID NO: 13 oder SEQ ID NO: 14 oder ist zu der Aminosäuresequenz der SEQ ID NO: 8, SEQ ID NO: 9, SEQ ID NO: 10, SEQ ID NO: 11, SEQ ID NO: 12, SEQ ID NO: 13 oder SEQ ID NO: 14 homolog. Die Transportproteine transportieren N-haltige Heterozyklen und gehören zu einer völlig neuartigen Klasse von Transportproteinen, die keinerlei Verwandtschaft zu dem aus Hefe bekannten Allantoin-Transporter DAL4 oder der PUP-Familie aus *Arabidopsis* aufweisen. Sie unterscheiden sich sowohl strukturell als auch im Substratspektrum deutlich von den bekannten Transportproteinen.

"Homolog" bedeutet hier, daß ein Polypeptid oder Protein eine signifikante Ähnlichkeit mit einer Vergleichssequenz aufweist. Homologe Sequenzen sind beispielsweise solche, die unter Zuhilfenahme des Similaritätsalgorithmus BLAST (Altschul et al., 1990, Basic local alignment search tool, J. Mol. Biol. 215, 403-410) eine signifikante Ähnlichkeit mit Vergleichssequenzen aufweisen. Als signifikant ähnlich werden hier Sequenzen verstanden, die beim Vergleich mit Vergleichssequenzen beispielsweise unter Verwendung des BLAST-Programms des National Centre for Biotechnology Information (NCBI) mit Standardparametern ein Signifikanzniveau p von < 10⁻⁵ aufweisen. Als homolog gelten des weiteren Sequenzen, die mit Vergleichssequenzen zu mindestens 70 %, bevorzugt zu 85 %, besonders bevorzugt zu über 95 % identisch sind.

Die vorliegende Erfindung betrifft auch ein Verfahren zur Herstellung einer transgenen Pflanze, umfassend die Schritte Einführen mindestens einer erfindungsgemäßen Nukleinsäure in eine Pflanzenzelle und Regenerieren einer Pflanze aus dieser Pflanzenzelle. Bevorzugt wird hierbei die Nukleinsäure in Antisense-Orientierung verwendet.

Mit Hilfe des Verfahrens sind Pflanzen erhältlich, die bezüglich des Im- und Exports sowie des Stoffwechsels von N-haltigen Heterozyklen gut kontrollierbar sind. Beispielsweise können nach dem erfindungsgemäßen Verfahren Pflanzen mit verstärkter Expression endogener Transporter N-haltiger Heterozyklen hergestellt werden. Insbesondere kann der Transport von Allantoin z.B. in Leguminosen und damit möglicherweise auch die Fixierungsrate von atmosphärischem Stickstoff beeinflußt werden. Diese Pflanzen können eine verbesserte N-Mobilisierung aufweisen. Es können aber auch Pflanzen hergestellt werden, deren endogene N-Heterozyklentransporter nicht, nur in geringem Maße oder nur in bestimmten Geweben exprimiert werden. So könnte beispielsweise bei der Sojabohne gezielt in das Öl/Protein-Verhältnis eingegriffen werden. Abhängig vom Verwendungszweck kann sowohl eine Verringerung als auch eine Steigerung des Protein-Gehaltes ein wesentliches Zuchtziel sein.

Darüber hinaus kann das erfindungsgemäße Verfahren aber auch eingesetzt werden, um die N-Heterozyklentransportereigenschaften einer Pflanze so zu beeinflussen, daß es zu einer Veränderungen der Spezifität des Transportsystems kommt, so daß der Transport neuer Verbindungen ermöglicht wird. Beispielsweise sind auch Veränderungen möglich, die die Affinität oder Substratspezifität des Transporters dahingehend verändern, daß es zu einem effizienteren Transport von N-haltigen Heterozyklen aus seneszenten Blättern kommt. Dies betrifft z.B. Alkaloide, die bisher von den Transportern nicht erkannt werden, synthetische Hormone, die schlecht transportiert werden und N-haltige Heterozyklen, die nicht effizient genug transportiert werden.

Außerdem kann die Verteilung von Pestiziden, die auf der chemischen Strukturgrundlage des Hydantoinringes aufgebaut sind (Marton et al., 1993, J. Agr. Food Chem. 41, 148-152; Sakai et al., 1993, J. Pest. Sci., 18, 217-223) dadurch beeinflußt werden. Auch der Einsatz neuer Pestizide bei Pflanzen mit veränderten N-Heterozyklentransportern wird hierdurch ermöglicht.

Die Erfindung betrifft auch die Verwendung einer erfindungsgemäßen Nukleinsäure zur Herstellung einer transgenen Pflanzenzelle oder Pflanze mit einer gegenüber dem Wildtyp erhöhten Expression der codierenden Region des Transportproteins für stickstoffhaltige Heterozyklen. Hierdurch sind Pflanzen erhältlich, die beispielsweise bestimmte Alkaloide stärker als Wildtyppflanzen anreichern.

Darüber hinaus betrifft die Erfindung die Verwendung einer erfindungsgemäßen Nukleinsäure in Antisense-Orientierung zur Herstellung einer transgenen Pflanzenzelle oder Pflanze mit einer gegenüber dem Wildtyp verminderten Expression der codierenden Region des Transportproteins für stickstoffhaltige Heterozyklen.

Die Erfindung wird im weiteren anhand der Figuren 1 bis 5 näher beschrieben. Es zeigt:
- Figur 1: (A) ein Schema zur Komplementation der dal4/dal5-Doppelmutante (Hefe) mit AtUPS1 sowie (B) Wachstum von komplementierten dal4/dal5-Doppelmutanten (dal4/dal5 UPS1) im Vergleich zu nicht-komplementierten Doppelmutanten (da14/da15) auf Medium mit Allantoin als alleiniger Stickstoffquelle.
- Figur 2: einen Vergleich der Allantoinaufnahmerate des mit einer erfindungsgemäßen Nukleinsäure transformierten Hefestamms GEM42::pFL61-UPS1 und des ohne die erfindungsgemäße Nukleinsäure transformierten Stamms GEM42::pFL61. Die Substratkonzentration (¹⁴C-Allantoin) betrug 200 mM.

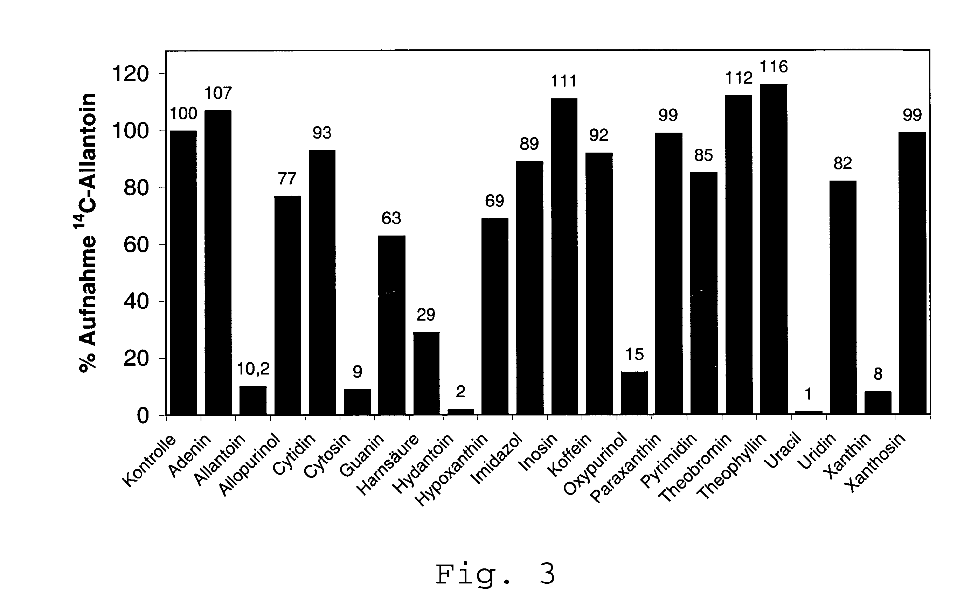

- Figur 3: eine Analyse der Substratspezifität des in Hefe exprimierten UPS1-N-Heterozyklentransporters. Angegeben ist die relative Aufnahme (100%) von ¹⁴C-markiertem Allantoin in Anwesenheit von unmarkiertem Adenin, Allantoin, Allopurinol, Cytidin, Cytosin, Guanin, Harnsäure, Hydantoin, Hypoxanthin, Imidazol, Inosin, Koffein, Oxypurinol, Paraxanthin, Pyrimidin, Theobromin, Theophyllin, Uracil, Uridin, Xanthin und Xanthosin. Die Konzentration von ¹⁴C-Allantoin betrug 0.2 mM, die der anderen Substanzen 2 mM. Werte < 100% weisen auf Verdrängung des Substrats ¹⁴C-Allantoin am Transportermolekül durch die jeweils andere Verbindung hin.
- Figur 4: Wachstum von mit UPS5b oder UPS1 komplementierten dal4/dal5-Doppelmutanten im Vergleich zu nichtkomplementierten Doppelmutanten (Vector) auf Medium mit Allantoin als alleiniger Stickstoffquelle.
- Figur 5: eine schematische Darstellung der angenommenen Struktur von UPS1.

Die Klonierung von N-Heterozyklentransportern erfolgte durch Komplementation von Hefemutanten. Mit Komplementation bezeichnet man eine sich im Phänotyp widerspiegelnde Kompensation eines genetischen Funktionsdefektes unter Beibehaltung der dem Defekt zugrundeliegenden Mutation(en). Komplementation liegt beispielsweise dann vor, wenn ein genetischer Defekt in einem Gen dazu führt, daß die Aufnahme einer für das Wachstum notwendigen Medienkomponente nicht mehr möglich ist und durch die Anwesenheit eines intakten Gens, das die Funktion des defekten Gens übernimmt, dieser Defekt aufgehoben wird, so daß nunmehr ein Wachstum wieder erfolgen kann.

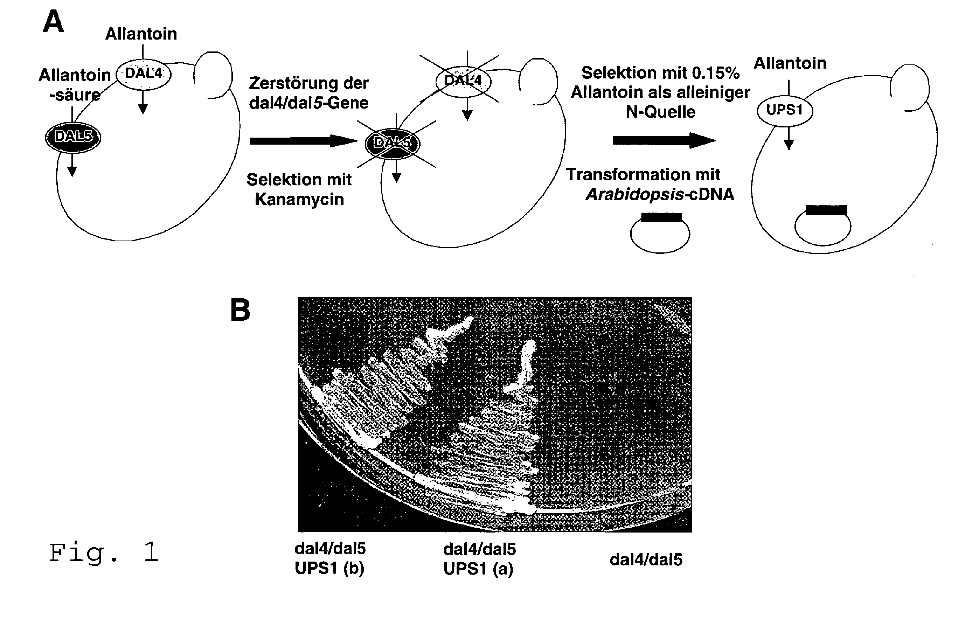

Zur Komplementation wurden in dem uracilauxotrophen Hefestamm S23344c (*Saccharomyces cerevisiae*) zunächst die Gene DAL4 und DAL5 durch homologe Rekombination ausgeschaltet (s. Fig. 1A). Diese dal4/dal5/ura3-Mutante, die im folgenden als dal4/dal5-Doppelmutante oder als GEM42 bezeichnet wird, ist nicht in der Lage, auf Medien, die Allantoin als einzige Stickstoffquelle enthalten, zu wachsen. GEM42 wurde mit cDNA aus jungen Keimlingen von *Arabidopsis thaliana* unter Verwendung des Hefe-Expressionsvektors pFL61 (Minet & Lacroute, 1990, Curr. Genet. 18, 287-291) nach der Methode von Dohmen et al., 1991, Yeast 7, 691-692, transformiert. Etwa 1 µg des Vektors mit einer cDNA-Insertion wurde hierfür verwendet. Hefetransformanten, die auf Minimalmedium mit 0.15% Allantoin als einziger Stickstoffquelle wachsen konnten, wurden vermehrt. Aus diesen Zellen wurde Plasmid-DNA nach Standardverfahren isoliert. Der Stamm GEM42 wurde erneut mit dem isolierten Plasmid transformiert. Auf diese Weise wurde ein Plasmid pFL61-UPS1 erhalten, das die dal4/dal5-Doppelmutation komplementieren konnte (s. Fig. 1B). Dieses Plasmid weist eine Insertion mit einer Größe von 1.2 kb auf, die das UPS1-N-Heterozyklentransportergen (SEQ ID NO: 1) enthält. Die Nukleinsäure gemäß SEQ ID NO: 1 codiert für ein Protein gemäß SEQ ID NO: 8. Für den entsprechenden N-Heterozyklentransporter wird im folgenden die Kurzbezeichnung UPS1 verwendet.

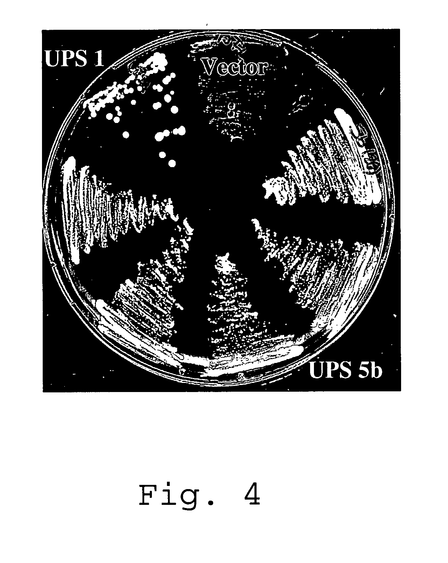

Auch die Gene gemäß SEQ ID NO 2 bis SEQ ID NO 7 verleihen der dal4/dal5-Mutante in diesem System die Eigenschaft, auf Allantoin als einziger N-Quelle wachsen zu können. In Fig. 4 ist dies für UPS5 (SEQ ID NO: 6 und 7) und PvUPS (SEQ ID NO: 5) dargestellt.

Der durch Transformation von GEM42 mit dem Plasmid pFL61-UPS1 erhaltene Hefestamm GEM42::pFL61-UPS1 wurde für Untersuchungen zur Aufnahme von Allantoin oder anderen N-haltigen Heterozyklen verwendet. Das N-Heterozyklentransportergen stand dabei unter der Kontrolle des Promotors der Phosphoglyceratkinase aus Hefe.

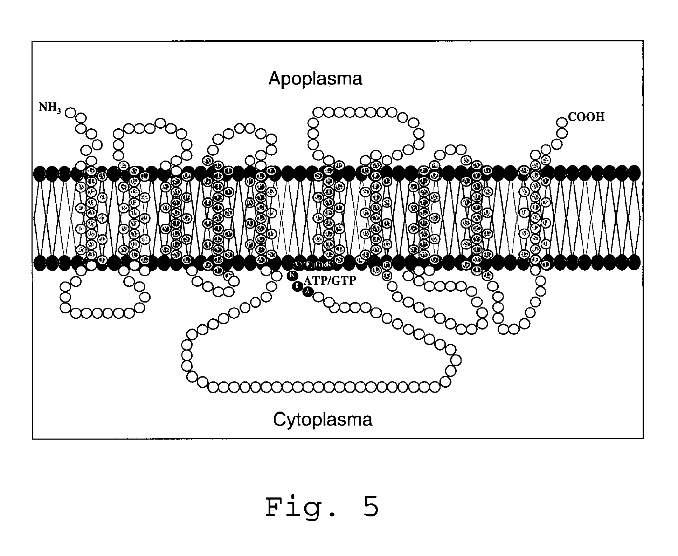

Das von dem Hefestamm GEM42::pFL61-UPS1 exprimierte UPS1-Protein wurde einer Hydrophobizitätsanalyse nach Kyte und Doolittle unterzogen. Das UPS1-Protein weist zehn stark hydrophobe Regionen auf, die lang genug sind, um jeweils einmal die Membran zu durchspannen (Fig. 5). Dies zeigt, daß UPS1 ein Membranprotein ist.

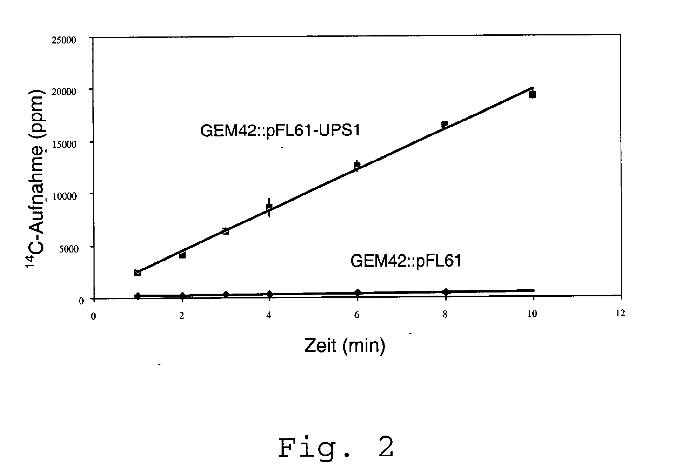

Um die Aufnahme von Allantoin durch GEM42::pFL61-UPS1 quantifizieren zu können, wurde ¹⁴C-markiertes Allantoin eingesetzt. Dieses wurde durch enzymatische Modifikation der verwandten Verbindung Harnsäure, die kommerziell als ¹⁴C-Derivat erhältlich ist, hergestellt. Hierzu wurde ¹⁴C-markierte Harnsäure mit hochreiner Uricase (Sigma) enzymatisch umgesetzt. Der Reinheitsgrad des erhaltenen Produkts ¹⁴C-Allantoin wurde mittels HPLC-Untersuchungen ermittelt. Die Aufnahme von ¹⁴C-Allantoin durch Hefezellen des Stamms GEM42, die mit der erfindungsgemäßen Nukleinsäure gemäß SEQ ID NO: 1, die für UPS1 codiert, transformiert wurden, ist in Fig. 2 dargestellt. Als Kontrolle wurden GEM42-Zellen verwendet, die lediglich mit dem Konstrukt ohne UPS1 transformiert wurden.

Für vergleichende Untersuchungen zu den Transportcharakteristiken der Transporterproteine wurden Kompetitionsstudien durchgeführt, wie sie prinzipiell bei Ninnemann et al., 1994, EMBO J. 15, 3464-3471, beschrieben sind. Hierzu wurde die Allantoinaufnahme in Gegenwart eines zehnfachen Überschusses eines anderen N-haltigen Heterozyklus (Adenin, Allopurinol, Cytidin, Cytosin, Guanin, Harnsäure, Hydantoin, Hypoxanthin, Imidazol, Inosin, Koffein, Oxypurinol, Paraxanthin, Pyrimidin, Theobromin, Theophyllin, Uracil, Uridin, Xanthin und Xanthosin) untersucht. Zur Kontrolle wurde auch die Allantoinaufnahme in Gegenwart eines zehnfachen Überschusses von unmarkiertem Allantoin ermittelt. Völlig unerwartet wurde hierbei festgestellt, daß andere N-haltige Heterozyklen, beispielsweise Harnsäure, Hydantoin, Cytosin, Oxypurinol, Uracil und Xanthin, ebenfalls von den Hefezellen aufgenommen wurden (s. Fig. 3). Mit Hilfe der erfindungsgemäßen Transporter nahmen die Hefezellen also neben Allantoin auch noch zahlreiche andere N-haltige Heterozyklen auf.

*Arabidopsis*-Pflanzen wurden mit einer Nukleinsäure gemäß der vorliegenden Erfindung transformiert. Die Transformation wurde mit Hilfe von *Agrobacterium tumefaciens* (Stamm C58C1, pGV2260) durchgeführt (Deblaere et al., 1985, Nucl. Acids Res. 13, 4777-4788). Der DNA-Transfer in die Agrobakterien erfolgte durch direkte Transformation nach der Methode von Höfgen und Willmitzer, 1988, Nucl. Acids Res. 16, 9877. Die Plasmid-DNA transformierter Agrobakterien wurde nach der Methode von Birnboim und Doly, 1979, Nucl. Acids Res. 7, 1513-1523, isoliert und nach geeigneter Restriktionsspaltung gelelektrophoretisch analysiert.

Die Transformation von *A*. *thaliana* wurde mittels Vakuum-Infiltration (modifiziert nach Bechtold et al., 1993, Comptes Rendus de l'Academie des Sciences Serie III, Sciences de la Vie 316: 1194-1199) ausgeführt. Töpfe (Durchmesser 10 cm) wurden mit Erde gefüllt und anschließend mit einem Fliegennetz überspannt. Auf diesem Netz wurden *A. thaliana-*Samen ausgesät. Sechs bis acht Wochen nach dem Aussäen wurden die Pflanzen für die Vakuum-Infiltration benutzt. Zur Vakuum-Infiltration wurden von den entsprechenden *Agrobacterium-*Stämmen 2 x 1 Liter Kulturen in YEB + Antibiotikum (50 µg/ml Kan und 100 µg/ml Rif) bei 28°C angezogen. Bei einer OD₆₀₀ von 1,5 wurden die Zellen bei 3000 g geerntet und in 600 ml Infiltrationsmedium (0,5 x MS Medium (Sigma), 5% Saccharose, 44 µM Benzylaminopurin) resuspendiert. Die Bakteriensuspension wurde in 250 ml Weckgläser gefüllt und in einen Exsikkator gestellt. Die *A. thaliana*-Pflanzen wurden "kopfüber" in die Bakteriensuspension getaucht, dann wurde für 5 min Vakuum angelegt. Nach 3-4 Wochen wurden die Samen dieser Pflanzen geerntet. Zur Oberflächensterilisation wurden die Samen 10 min lang in 4% Natriumhypochlorid, 0,02% Triton geschüttelt, bei 1500 g abzentrifugiert, viermal mit sterilem Wasser gewaschen und in 3 ml 0.05% Agarose pro 5000 Samen resuspendiert. Die Samen-Agarose-Lösung wurde auf MSS Medium (1 x MS, 1% Saccharose, 0,8% Agarose, 50 µg/ml Kanamycin, pH 5,8) ausgebreitet (Platten mit 13,5 cm Durchmesser für 5000 Samen). Zur Reduzierung des Feuchtigkeitsverlustes wurden die Platten mit Parafilm verschlossen. Die kanamycinresistenten Pflanzen wurden in Erde umgesetzt. Samen dieser Pflanzen wurden geerntet und analysiert.

## Patentansprüche

1. Nukleinsäure, die die codierende Region für ein Transportprotein für stickstoffhaltige Heterozyklen, insbesondere deren Oxoderivate, enthält, **dadurch gekennzeichnet, daß** die Nukleinsäure für ein Transportprotein codiert, das eine oder mehrere Verbindungen, ausgewählt aus der Gruppe bestehend aus
a) einem Oxoderivat mit mindestens zwei Oxo-Gruppen im Ringsystem des Heterozyklus, ausgenommen Koffein
b) einem Oxoderivat mit mindestens drei Oxo-Gruppen im Ringsystem des Heterozyklus
transportiert.

2. Nukleinsäure nach Anspruch 1, **dadurch gekennzeichnet, daß** es sich
a) bei dem Oxoderivat mit mindestens zwei Oxo-Gruppen im Ringsystem des Heterozyklus um Hydantoin, Oxypurinol, Uracil oder Xanthin,
b) bei dem Oxoderivat mit mindestens drei Oxo-Gruppen im Ringsystem des Heterozyklus um Allantoin oder Urat
handelt.

3. Nukleinsäure, die die codierende Region für ein Transportprotein für stickstoffhaltige Heterozyklen, insbesondere deren Oxoderivate, enthält, **dadurch gekennzeichnet, daß** die Nukleinsäure
a) eine DNA-Sequenz, die für ein Peptid gemäß einer der Sequenzen der SEQ ID NO: 15 bis SEQ ID NO: 22 codiert, oder
b) eine zu der DNA-Sequenz gemäß a) komplementäre Nukleotidsequenz, oder
c) eine der DNA-Sequenz gemäß a) entsprechende RNA-Sequenz oder DNA/RNA-Mischsequenz umfaßt, oder
d) mit einer Nukleinsäure, die die DNA-Sequenz gemäß a) oder eine zu der DNA-Sequenz gemäß a) komplementäre Nukleotidsequenz aufweist, hybridisiert.

4. Nukleinsäure nach Anspruch 3, **dadurch gekennzeichnet, daß** die Nukleinsäure
a) eine DNA-Sequenz der SEQ ID NO: 1, SEQ ID NO: 2, SEQ ID NO: 3, SEQ ID NO: 4, SEQ ID NO: 5, SEQ ID NO: 6 oder SEQ ID NO: 7, oder
b) eine zu der DNA-Sequenz gemäß a) komplementäre Nukleotidsequenz, oder
c) eine der DNA-Sequenz gemäß a) entsprechende RNA-Sequenz oder DNA/RNA-Mischsequenz umfaßt, oder
d) mit einer Nukleinsäure, die die DNA-Sequenz gemäß a) oder eine zu der DNA-Sequenz gemäß a) komplementäre Nukleotidsequenz aufweist, hybridisiert.

5. Nukleinsäurekonstrukt, Vektor oder mobiles genetisches Element, enthaltend mindestens eine Nukleinsäure gemäß einem der Ansprüche 1 bis 4.

6. Nukleinsäurekonstrukt, Vektor oder mobiles genetisches Element nach Anspruch 5, **dadurch gekennzeichnet, daß** die Nukleinsäure in Antisense-Orientierung enthalten ist.

7. Eukaryotische oder prokaryotische Wirtszelle, enthaltend mindestens eine Nukleinsäure gemäß einem der Ansprüche 1 bis 4.

8. Eukaryotische oder prokaryotische Wirtszelle nach Anspruch 7, **dadurch gekennzeichnet, daß** die Nukleinsäure in Antisense-Orientierung enthalten ist.

9. Pflanze oder Teile davon, transformiert mit mindestens einer Nukleinsäure gemäß einem der Ansprüche 1 bis 4.

10. Transgene Pflanze nach Anspruch 9, **dadurch gekennzeichnet, daß** die Pflanze mit der Nukleinsäure in Antisense-Orientierung transformiert ist.

11. Transgene Samen von Pflanzen nach einem der Ansprüche 9 oder 10.

12. Transportprotein für stickstoffhaltige Heterozyklen, insbesondere deren Oxoderivate, **dadurch gekennzeichnet, daß** das Transportprotein eine oder mehrere Verbindungen, ausgewählt aus der Gruppe bestehend aus
a) einem Oxoderivat mit mindestens zwei Oxo-Gruppen im Ringsystem des Heterozyklus, ausgenommen Koffein
b) einem Oxoderivat mit mindestens drei Oxo-Gruppen im Ringsystem des Heterozyklus
transportiert.

13. Transportprotein nach Anspruch 12, **dadurch gekennzeichnet, daß** es sich
a) bei dem Oxoderivat mit mindestens zwei Oxo-Gruppen im Ringsystem des Heterozyklus um Hydantoin, Oxypurinol, Uracil oder Xanthin,
b) bei dem Oxoderivat mit mindestens drei Oxo-Gruppen im Ringsystem des Heterozyklus um Allantoin oder Urat
handelt.

14. Transportprotein für stickstoffhaltige Heterozyklen, insbesondere deren Oxoderivate, **dadurch gekennzeichnet, daß** die Aminosäuresequenz des Transportproteins
a) die Aminosäuresequenz der SEQ ID NO: 15, SEQ ID NO: 16, SEQ ID NO: 17, SEQ ID NO: 18, SEQ ID NO: 19, SEQ ID NO: 20, SEQ ID NO: 21 oder SEQ ID NO: 22, oder
b) eine zu der Aminosäuresequenz gemäß a) homologe Aminosäuresequenz umfaßt.

15. Transportprotein nach Anspruch 14, **dadurch gekennzeichnet, daß** die Aminosäuresequenz des Transportproteins
a) die Aminosäuresequenz der SEQ ID NO: 8, SEQ ID NO: 9, SEQ ID NO: 10, SEQ ID NO: 11, SEQ ID NO: 12, SEQ ID NO: 13 oder SEQ ID NO: 14 oder
b) eine zu der Aminosäuresequenz gemäß a) homologe Aminosäuresequenz umfaßt.

16. Verfahren zur Herstellung einer transgenen Pflanze, umfassend die Schritte
Einführen mindestens einer Nukleinsäure gemäß einem der Ansprüche 1 bis 4 in eine Pflanzenzelle und
Regenerieren einer Pflanze aus dieser Pflanzenzelle.

17. Verfahren nach Anspruch 16, **dadurch gekennzeichnet, daß** die Nukleinsäure in Antisense-Orientierung verwendet wird.

18. Verwendung einer Nukleinsäure nach einem der Ansprüche 1 bis 4 zur Herstellung einer transgenen Pflanzenzelle oder Pflanze mit einer gegenüber dem Wildtyp erhöhten Expression der codierenden Region des Transportproteins für stickstoffhaltige Heterozyklen.

19. Verwendung einer Nukleinsäure nach einem der Ansprüche 1 bis 4 in Antisense-Orientierung zur Herstellung einer transgenen Pflanzenzelle oder Pflanze mit einer gegenüber dem Wildtyp verminderten Expression der codierenden Region des Transportproteins für stickstoffhaltige Heterozyklen.
